# EUROPEAN PATENT SPECIFICATION

(11) **EP 2 152 290 B1**
(45) Date of publication and mention of the grant of the patent: **04.06.2014**
(21) Application number: 08747188.4
(22) Date of filing: 30.04.2008
(51) Int. Cl.: A61K 38/10

(54) **METHODS FOR ADMINISTERING ANTI-IL-5 ANTIBODIES**
VERFAHREN ZUR VERABREICHUNG VON ANTI-IL-5-ANTIKÖRPERN
MÉTHODES POUR ADMINISTRER DES ANTICORPS ANTI-IL-5

(30) Priority: 30.04.2007 US 914833 P; 28.11.2007 US 990715 P
(43) Date of publication of application: 17.02.2010
(60) Divisional application: 13152633.7
(73) Proprietor: GlaxoSmithKline LLC, Wilmington, Delaware 19808 (US)
(72) Inventor: PATEL, Bela, Rajiv, King Of Prussia, PA 19406 (US); SMITH, Deborah, Durham, NC 27709 (US); TOMPSON, Debra, J., Harlow, Essex CM19 5AW (GB); ZIA-AMIRHOSSEINI, Parnian, Thousand Oaks, CA 91320 (US)
(74) Representative: Harrison, Anna
(86) International application number: PCT/US2008/062015
(87) International publication number: WO 2008/134721

(56) References cited:
- US-A1- 2003 059 429
- US-A1- 2003 194 404
- HART TIMOTHY K ET AL: "Preclinical efficacy and safety of mepolizumab (SB-240563), a humanized monoclonal antibody to IL-5, in cynomolgus monkeys" August 2001 (2001-08), JOURNAL OF ALLERGY AND CLINICAL IMMUNOLOGY, VOL. 108, NR. 2, PAGE(S) 250-257 , XP002577858 ISSN: 0091-6749 * abstract; figure 3 *
- ZIA-AMIRHOSSEINI P ET AL: "Pharmacokinetics and pharmacodynamics of SB-240563, a humanized monoclonal antibody directed to human interleukin-5, in monkeys" JOURNAL OF PHARMACOLOGY AND EXPERIMENTAL THERAPEUTICS, vol. 291, no. 3, December 1999 (1999-12), pages 1060-1067, XP002577859 ISSN: 0022-3565
- ZIA-AMIRHOSSEINI ET AL: "Pharmacokinetics and pharmacodynamics of SB-240563, a humanized monoclonal antibody directed to human interleukin-5, in mild asthmatics" CLINICAL PHARMACOLOGY AND THERAPEUTICS, NATURE PUBLISHING GROUP, US LNKD- DOI:10.1016/S0009-9236(99)80120-9, vol. 65, no. 2, 1 February 1999 (1999-02-01), page 147, XP005467519 ISSN: 0009-9236
- LECKIE ET AL: "Effects of an interleukin-5 blocking monoclonal antibody on eosinophils, airway hyper-responsiveness, and the late asthmatic response" LANCET THE, LANCET LIMITED. LONDON, GB LNKD- DOI:10.1016/S0140-6736(00)03496-6, vol. 356, no. 9248, 23 December 2000 (2000-12-23), pages 2144-2148, XP005071953 ISSN: 0140-6736
- GNANAKUMARAN GNANASEGARAM ET AL: "Technology evaluation: mepolizumab, GlaxoSmithKline" CURRENT OPINION IN MOLECULAR THERAPEUTICS, CURRENT DRUGS, LONDON, GB, vol. 5, no. 3, 1 June 2003 (2003-06-01), pages 321-325, XP009132121 ISSN: 1464-8431
- REVILL P ET AL: "Mepolizumab. Humanized anti-IL-5 monoclonal antibody, Treatment of hypereosinophilic syndromes" DRUGS OF THE FUTURE, vol. 32, no. 10, October 2007 (2007-10), pages 868-873, XP002577860 ISSN: 0377-8282

### SEQUENCE LISTING.TXT

<110> PATEL, Bela Rajiv
   SMITH, Deborah
   TOMPSON, Debra J.
   ZIA-AMIRHOSSEINI, Parnian
<120> METHODS FOR ADMINISTERING ANTI-IL-5 ANTIBODIES
<130> PU62439A
<150> PCT/US2008/062015
   <151> 2008-04-30
<150> US 60/914833
   <151> 2007-04-30
<150> US 60/990715
   <151> 2007-11-28
<160> 21
<170> FastSEQ for windows version 4.0
<210> 1
   <211> 334
   <212> DNA
   <213> Murine
<400> 1
   acctggcctg gtggcgccct cacagagcct gtccatcact tgcactgtct ctgggttttc 60
   attaaccagc tatagtgtac actgggttcg ccagcctcca ggaaagggtc tggagtggct 120
   gggagtaata tgggctagtg gaggcacaga ttataattcg gctctcatgt ccagactgag 180
   catcagcaaa gacaactcca agagccaagt tttcttaaaa ctgaacagtc tgcaaactga 240
   tgacacagcc atgtactact gtgccagaga tcccccttct tccttactac ggcttgacta 300
   ctggggccaa ggcaccactc tcacagtctc ctca 334
<210> 2
   <211> 315
   <212> DNA
   <213> Murine
<400> 2
   tcctccctga gtgtgtcagc aggagagaag gtcactatga gctgcaagtc cagtcagagt 60
   ctgttaaaca gtggaaatca aaagaactac ttggcctggt accagcagaa accagggcag 120
   cctcctaaac ttttgatcta cggggcatcc actagggaat ctggggtccc tgatcgcttc 180
   acaggcagtg gatctggaac cgatttcact ctttccatca gcagtgtgca ggctgaagac 240
   ctggcagttt attactgtca gaatgttcat agttttccat tcacgttcgg ctcggggaca 300
   gagttggaaa taaaa                                                  315
<210> 3
   <211> 334
   <212> DNA
   <213> Murine
<400> 3
   acctggcctg gtggcgccct cacagagcct gtccatcact tgcactgtct ctgggttttc 60
   attaaccagt tatagtgtac actgggttcg ccagcctcca ggaaagggtc tggagtggct 120
   gggagtaata tgggctagtg gaggcacaga ttataattcg gctctcatgt ccagactgag 180
   catcagcaaa gacaactcca agagccaagt tttcttaaaa ctgaacagtc tgcgaactga 240
   tgacacagcc atgtactact gtgccagaga tcccccttct tccttactac ggcttgacta 300
   ctggggccaa ggcaccactc tcacagtctc ctca                             334
<210> 4
   <211> 315
   <212> DNA
   <213> Murine
<400> 4
   tcctccctga gtgtgtcagc aggagagaag gtcactatga gctgcaagtc cagtcagagt 60
   ctattaaaca gtggaaatca aaagaactac ttggcctggt accaacagaa accagggcag 120
   cctcctaaac ttttgatcta cggggcatcc actagggaat ctggggtccc tgatcgcttc 180
   acaggcagtg gatctggaac cgatttcact cttaccatca gcagtgtgca ggctgaagac 240
   ctggcagttt attactgtca gaatgatcat agttttccat tcacgttcgg ctcggggaca 300
   gagttggaaa taaaa                                                  315
<210> 5
   <211> 334
   <212> DNA
   <213> Murine
<400> 5
   acctggcctg gtggcgccct cacagagcct gtccatcact tgcactgtct ctgggttttc 60
   attaaccagc tatagtgtac actgggttcg ccagcctcca ggaaagggtc tggagtggct 120
   gggagtaatc tgggctagtg gaggcacaga ttataattcg gctctcatgt ccagactgag 180
   catcagcaaa gacaactcca agagccaagt tttcttaaaa ctgaacagtc tgcaaactga 240
   tgacgcagcc atgtactact gtgccagaga tccccctttt tccttactac ggcttgactt 300
   ctggggccaa ggcaccactc tcacagtctc ctca                             334
<210> 6
   <211> 315
   <212> DNA
   <213> Murine
<400> 6
   tcctctctga gtgtgtcagc aggagagaag gtcactatga gctgcaagtc cagtcagagt 60
   ctgttaaaca gtggaaatca aaaaaactac ttggcctggt accagcagaa accagggcag 120
   cctcctaaac ttttgatcta cggggcatcc actagggaat ctggggtccc tgatcgcttc 180
   acaggcagtg gatctggaac cgatttcact cttaccatca gcagtgtgca ggctgaagac 240
   ctggcagttt attactgtca gaatgatcat agttttccat tcacgttcgg ctcggggaca 300
   gagttggaaa taaaa                                                  315
<210> 7
   <211> 5
   <212> PRT
   <213> Murine
<400> 7
   Ser Tyr Ser Val His
   1               5
<210> 8
   <211> 16
   <212> PRT
   <213> Murine
<400> 8
   Val Ile Trp Ala Ser Gly Gly Thr Asp Tyr Asn Ser Ala Leu Met Ser
   1               5                   10                  15
<210> 9
   <211> 11
   <212> PRT
   <213> Murine
<400> 9
   Asp Pro Pro Ser Ser Leu Leu Arg Leu Asp Tyr
   1               5                   10
<210> 10
   <211> 17
   <212> PRT
   <213> Murine
<400> 10
   Lys Ser Ser Gln Ser Leu Leu Asn Ser Gly Asn Gln Lys Asn Tyr Leu
   1               5                   10                  15
   Ala
<210> 11
   <211> 7
   <212> PRT
   <213> Murine
<400> 11
   Gly Ala Ser Thr Arg Glu Ser
   1               5
<210> 12
   <211> 9
   <212> PRT
   <213> Murine
<400> 12
   Gln Asn Val His Ser Phe Pro Phe Thr
   1 5
<210> 13
   <211> 9
   <212> PRT
   <213> Murine
<400> 13
   Gln Asn Asp His Ser Phe Pro Phe Thr
   1 5
<210> 14
   <211> 11
   <212> PRT
   <213> Murine
<400> 14
   Asp Pro Pro Phe Ser Leu Leu Arg Leu Asp Phe
   1 5 10
<210> 15
   <211> 119
   <212> PRT
   <213> Artificial Sequence
<220>
   <223> Murine/Homo sapiens
<400> 15
   Gln Val Gln Leu Lys Glu Ser Gly Pro Gly Leu Val Ala Pro Ser Gln
   1 5 10 15
   Ser Leu Ser Ile Thr Cys Thr Val Ser Gly Phe Ser Leu Thr Ser Tyr
   20 25 30
   Ser Val His Trp Val Arg Gln Pro Pro Gly Lys Gly Leu Glu Trp Leu
   35 40 45
   Gly Val Ile Trp Ala Ser Gly Gly Thr Asp Tyr Asn Ser Ala Leu Met
   50 55 60
   Ser Arg Leu Ser Ile Ser Lys Asp Asn Ser Lys Ser Gln Val Phe Leu
   65 70 75 80
   Lys Leu Asn Ser Leu Gln Thr Asp Asp Thr Ala Met Tyr Tyr Cys Ala
   85 90 95
   Arg Asp Pro Pro Ser Ser Leu Leu Arg Leu Asp Tyr Trp Gly Gln Gly
   100 105 110
   Thr Thr Leu Thr Val Ser Ser
   115
<210> 16
   <211> 113
   <212> PRT
   <213> Artificial sequence
<220>
   <223> Murine/Homo sapiens
<400> 16
   Asp Ile Val Met Thr Gln Ser Pro Ser Ser Leu Ser Val Ser Ala Gly
    1               5                  10                  15
   Glu Lys Val Thr Met Ser Cys Lys Ser Ser Gln Ser Leu Leu Asn Ser
               20                  25                  30
   Gly Asn Gln Lys Asn Tyr Leu Ala Trp Tyr Gln Gln Lys Pro Gly Gln
           35                  40                  45
   Pro Pro Lys Leu Leu Ile Tyr Gly Ala Ser Thr Arg Glu Ser Gly Val
       50                  55                  60
   Pro Asp Arg Phe Thr Gly Ser Gly Ser Gly Thr Asp Phe Thr Leu Ser
   65                  70                  75                  80
   Ile Ser Ser Val Gln Ala Glu Asp Leu Ala Val Tyr Tyr Cys Gln Asn
                   85                  90                  95
   Val His Ser Phe Pro Phe Thr Phe Gly Ser Gly Thr Glu Leu Glu Ile
               100                 105                 110
   Lys
<210> 17
   <211> 60
   <212> DNA
   <213> Homo sapiens
<400> 17
   atggtgttgc agacccaggt cttcatttct ctgttgctct ggatctctgg tgcctacggg 60
<210> 18
   <211> 357
   <212> DNA
   <213> Artificial Sequence
<220>
   <223> Murine/Homo sapiens - humanized
<400> 18
   caggttaccc tgcgtgaatc cggtccggca ctagttaaac cgacccagac cctgacgtta 60
   acctgcaccg tctccggttt ctccctgacg agctatagtg tacactgggt ccgtcagccg 120
   ccgggtaaag gtctagaatg gctgggtgta atatgggcta gtggaggcac agattataat 180
   tcggctctca tgtcccgtct gtcgatatcc aaagacacct cccgtaacca ggttgttctg 240
   accatgacta acatggaccc ggttgacacc gctacctact actgcgctcg agatccccct 300
   tcttccttac tacggcttga ctactggggt cgtggtaccc cagttaccgt gagctca    357
<210> 19
   <211> 119
   <212> PRT
   <213> Artificial Sequence
<220>
   <223> Murine/Homo sapiens - humanized
<400> 19
<210> 20
   <211> 339
   <212> DNA
   <213> Artificial sequence
<220>
   <223> Murine/Homo sapiens - humanized
<400> 20
   gatatcgtga tgacccagtc tccagactcg ctagctgtgt ctctgggcga gagggccacc 60
   atcaactgca agagctctca gagtctgtta aacagtggaa atcaaaagaa ctacttggcc 120
   tggtatcagc agaaacccgg gcagcctcct aagttgctca tttacggggc gtcgactagg 180
   gaatctgggg tacctgaccg attcagtggc agcgggtctg ggacagattt cactctcacc 240
   atcagcagcc tgcaggctga agatgtggca gtatactact gtcagaatgt tcatagtttt 300
   ccattcacgt tcggcggagg gaccaagttg gagatcaaa                        339
<210> 21
   <211> 113
   <212> PRT
   <213> Artificial sequence
<220>
   <223> Murine/Homo sapiens - humanized
<400> 21

## Description

### Field of the Invention

The present invention relates generally to the methods for the treatment of conditions mediated by IL-5 and excess eosinophil production and methods of administering compounds for the treatment of these conditions.

### Background of the Invention

Eosinophils have been implicated in the pathogenesis of a wide variety of inflammatory disease states including allergic disorders associated with hypersensitivity reactions in the lung tissue (Butterfield et al., In: Immunopharmacology of Eosinophils, H. Smith and R. Cook, Eds., p.151-192, Academic Press, London (1993)). A notable example is asthma, a disease characterized by reversible obstruction of the airways leading to non-specific bronchial hyperresponsiveness. This obstruction in turn is dependent upon the generation of a chronic inflammatory reaction at the level of the bronchial mucosa and a characteristic infiltration by macrophages, lymphocytes and eosinophils. The eosinophil appears to play a central role in initiating the mucosal damage typical of the disease (Corrigan et al., Immunol. Today, 13:501-507 (1992)). Increased numbers of activated eosinophils have been reported in the circulation, bronchial secretions and lung parenchyma of patients with chronic asthma, and the severity of the disease, as measured by a variety of lung function tests, correlates with blood eosinophil numbers (Griffen et al., J. Aller. Clin. Immunol., 67:548-557 (1991)). Increased numbers of eosinophils, often in the process of degranulation, have also been recovered in bronchoalveolar lavage (BAL) fluids of patients undergoing late asthmatic reactions, and reducing eosinophil numbers, usually as a consequence of steroid therapy, is associated with improvements in clinical symptoms (Bousquet et al., N. Eng. J. Med., 323:1033-1039 (1990)).

Interleukin 5 (IL-5) is a homodimeric glycoprotein produced predominantly by activated CD4+ T lymphocytes. In man, IL-5 is largely responsible for controlling the growth and differentiation of eosinophils. Elevated levels of IL-5 are detected in the bronchoalveolar lavage washings of asthmatics (Motojima et al., Allergy, 48:98 (1993)). Mice which are transgenic for IL-5 show a marked eosinophilia in peripheral blood and tissues in the absence of antigenic stimulation (Dent et al., J. Exp. Med., 172:1425 (1990)) and anti-murine IL-5 monoclonal antibodies have been shown to have an effect in reducing eosinophilia in the blood and tissues of mice (Hitoshi et al., Int. Immunol., 3:135 (1991)) as well as the eosinophilia associated with parasite infection and allergen challenge in experimental animals (Coffman et al., Science, 245:308-310 (1989), Sher et al., Proc. Natl. Acad. Sci., 83:61-65 (1990), Chand et al., Eur. J. Pharmacol., 211:121-123 (1992)).

Although corticosteroids are extremely effective in suppressing eosinophil numbers and other inflammatory components of asthma, there are concerns about their side effects in both severe asthmatics and more recently in mild to moderate asthmatics. The only other major antiinflammatory drug therapies - cromoglycates (cromolyn sodium and nedocromil) - are considerably less effective than corticosteroids and their precise mechanism of action remains unknown.

The primary beneficial function of eosinophils is considered to be host protection against parasitic helminth infections (Klion AD, Nutman TB. J Allergy Clin Immunol 2004; 113: 30-7) and possibly some viruses (Rothenberg ME, Hogan SP. Annu Rev Immunol 2006; 24: 147-74). Parasitic invasion prompts rapid recruitment of eosinophils from the circulation to the site of infection, where they initiate and advance the immune response through a variety of mechanisms, including antigen presentation, and the secretion of proinflammatory cytokines, chemokines, lipid mediators and cytotoxic granule proteins (Kariyawasam HH, Robinson DS. Semin Respir Crit Care Med 2006; 27: 117-27 and Klion, et al.). Allograft and tumour antigens may also activate eosinophils and trigger degranulation, suggesting a potential role for eosinophils in organ transplant rejection and defence against malignant tumours (Munitz A, Levi-Schaffer F. Allergy 2004; 59: 268-75).

Hart et al., Journal of Allergy and Clinical Immunology, 2001, 108(2), 250-257 relates to the preclinical efficacy and safety of mepolizumab (SB-240563) in cynomolgus monkeys.

Zia-Amirhosseini, et al., Journal of Pharmacology and Experimental Therapeutics, 1999, 291 (3), 1060-1067 relates to the pharmacokinetics and pharmacodynamics of mepolizumab (SB-240563) in monkeys.

Zia-Amirhosseini, et al., Clinical Pharmacology and Therapeutics, 1999, 65(2), 147 relates to the pharmacokinetics and pharmacodynamics of mepolizumab (SB-240563) in mild asthmatics.

Leckie et al., The Lancet, 2000, 356(9248), 2144-2148 relates to the effects of mepolizumab (SB-240563) on eosinophils, airways hyper-responsiveness and the late asthmatic response.

Gnanakumaran Gnanasegaram et al., Current Opinion in Molecular Therapeutics, 2003, 5(3), 321-325 relates to a technology evaluation of mepolizumab (SB-240563).

Eosinophils have been implicated in a number of disease pathologies. Inappropriate secretion of cytokines and other effector molecules from eosinophils causes damage and dysfunction to the surrounding tissue. End-organ damage resulting from eosinophil infiltration and activation represents a common pathogenic component of several disease states, including atopic diseases and hypereosinophilic syndromes (HES). Thus, there is a need for the methods of the present invention and disclosure to reduce eosinophils in a human in need thereof.

### Summary of the invention

In one embodiment of the present invention and disclosure, methods are provided for reducing eosinophils in a human in need thereof, which method comprises administering to said human a composition comprising at least one anti-IL-5 antibody, wherein said anti-IL-5 antibody provides a mean maximum plasma concentration of said anti-IL-5 antibody of at least about 1.03 ± 0.21 µg/mL and an Area Under the Curve value of said anti-IL-5 antibody is at least about 15.5 ± 2.7 µg×day/mL.

In another embodiment, methods are provided for treating nasal polyposis, in a human, comprising the step of administering to said human in need thereof an effective amount of a composition comprising at least one anti-IL-5 antibody wherein said antibody comprises a heavy chain and a light chain.

### Detailed Description of the Invention

### I. Definitions.

As used herein an "anti-IL-5 antibody" refers to any antibody, antibody fragment or single chain antibody that binds to the cytokine IL-5 from any species. An anti-IL-5 antibody may be murine, chimeric, humanized or fully human. The antibody may be neutralizing. Several examples of anti-IL-5 antibodies are described in U.S. Patent Nos. 5,683,892, 5,693,323, 5,783,184, 5,851,525, 6,129,913, 5,096,071, 6,056,957, and 6,451,982. In addition, humanized anti-IL-5 antibodies are described in various references and include relizumab (SCH55700) and mepolizumab (SB240563) (Greenfeder, et al., Respiratory Research, 2(2):71-79 (2001)). Mepolizumab (SB-240563) is a fully humanized monoclonal antibody (IgG₁, kappa, mAb) which is specific for human interleukin-5 (IL-5).

"Altered antibody" refers to a protein encoded by an altered immunoglobulin coding region, which may be obtained by expression in a selected host cell. Such altered antibodies are engineered antibodies (e.g., chimeric or humanized antibodies) or antibody fragments lacking all or part of an immunoglobulin constant region, e.g., Fv, Fab, or F(ab)₂ and the like.

"Altered immunoglobulin coding region" refers to a nucleic acid sequence encoding an altered antibody of the invention and/or disclosure. When the altered antibody is a CDR-grafted or humanized antibody, the sequences that encode the complementarity determining regions (CDRs) from a non-human immunoglobulin are inserted into a first immunoglobulin partner comprising human variable framework sequences. Optionally, the first immunoglobulin partner is operatively linked to a second immunoglobulin partner.

"First immunoglobulin partner" refers to a nucleic acid sequence encoding a human framework or human immunoglobulin variable region in which the native (or naturally-occurring) CDR-encoding regions are replaced by the CDR-encoding regions of a donor antibody. The human variable region can be an immunoglobulin heavy chain, a light chain (or both chains), an analog or functional fragments thereof. Such CDR regions, located within the variable region of antibodies (immunoglobulins) can be determined by known methods in the art. For example Kabat et al. (Sequences of Proteins of Immunological Interest, 4th Ed., U.S. Department of Health and Human Services, National Institutes of Health (1987)) disclose rules for locating CDRs. In addition, computer programs are known which are useful for identifying CDR regions/structures.

"Neutralizing" refers to an antibody that inhibits IL-5 activity by preventing the binding of human IL-5 to its specific receptor or by inhibiting the signalling of IL-5 through its receptor, should binding occur. A mAb is neutralizing if it is 90% effective, preferably 95% effective and most preferably 100% effective in inhibiting IL-5 activity as measured in the B13 cell bioassay (IL-5 Neutralization assay, see Example 2C).

The term "high affinity" refers to an antibody having a binding affinity characterized by a K*_{d}* equal to or less than 3.5 x 10⁻¹¹ M for human IL-5 as determined by optical biosensor anaylsis.

By "binding specificity for human IL-5" is meant a high affinity for human, not murine, IL-5.

"Second immunoglobulin partner" refers to another nucleotide sequence encoding a protein or peptide to which the first immunoglobulin partner is fused in frame or by means of an optional conventional linker sequence (i.e., operatively linked). Preferably it is an immunoglobulin gene. The second immunoglobulin partner may include a nucleic acid sequence encoding the entire constant region for the same (i.e., homologous - the first and second altered antibodies are derived from the same source) or an additional (i.e., heterologous) antibody of interest. It may be an immunoglobulin heavy chain or light chain (or both chains as part of a single polypeptide). The second immunoglobulin partner is not limited to a particular immunoglobulin class or isotype. In addition, the second immunoglobulin partner may comprise part of an immunoglobulin constant region, such as found in a Fab, or F(ab)₂ (i.e., a discrete part of an appropriate human constant region or framework region). Such second immunoglobulin partner may also comprise a sequence encoding an integral membrane protein exposed on the outer surface of a host cell, e.g., as part of a phage display library, or a sequence encoding a protein for analytical or diagnostic detection, e.g., horseradish peroxidase, β-galactosidase, etc.

The terms Fv, Fc, Fd, Fab, or F(ab)₂ are used with their standard meanings (see, e.g., Harlow et al., Antibodies A Laboratory Manual, Cold Spring Harbor Laboratory, (1988)).

As used herein, an "engineered antibody" describes a type of altered antibody, i.e., a full-length synthetic antibody (e.g., a chimeric or humanized antibody as opposed to an antibody fragment) in which a portion of the light and/or heavy chain variable domains of a selected acceptor antibody are replaced by analogous parts from one or more donor antibodies which have specificity for the selected epitope. For example, such molecules may include antibodies characterized by a humanized heavy chain associated with an unmodified light chain (or chimeric light chain), or vice versa. Engineered antibodies may also be characterized by alteration of the nucleic acid sequences encoding the acceptor antibody light and/or heavy variable domain framework regions in order to retain donor antibody binding specificity. These antibodies can comprise replacement of one or more CDRs (preferably all) from the acceptor antibody with CDRs from a donor antibody described herein.

A "chimeric antibody" refers to a type of engineered antibody which contains naturally-occurring variable region (light chain and heavy chains) derived from a donor antibody in association with light and heavy chain constant regions derived from an acceptor antibody.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity (see, e.g., Queen et al., Proc. Natl Acad Sci USA, 86:10029-10032 (1989), Hodgson et al., Bio/Technology, 9:421 (1991)).

The term "donor antibody" refers to an antibody (monoclonal, or recombinant) which contributes the nucleic acid sequences of its variable regions, CDRs, or other functional fragments or analogs thereof to a first immunoglobulin partner, so as to provide the altered immunoglobulin coding region and resulting expressed altered antibody with the antigenic specificity and neutralizing activity characteristic of the donor antibody. One suitable donor antibody is a non-human neutralizing monoclonal antibody (i.e., murine) designated as 2B6, which was deposited with the American Type Culture Collection (ATCC), Rockville, MD, USA, under accession number HB 11783. The antibody 2B6 is defined as a high affinity, human-IL-5 specific (i.e., does not recognize murine IL-5), neutralizing antibody of isotype IgG₁ having the variable light chain DNA and amino acid sequences of SEQ ID NOs: 2 and 16, respectively, and the variable heavy chain DNA and amino acid sequences of SEQ ID NOs: 1 and 15, respectively, on a suitable murine IgG constant region.

The term "acceptor antibody" refers to an antibody (monoclonal, or recombinant) heterologous to the donor antibody, which contributes all (or any portion, but preferably all) of the nucleic acid sequences encoding its heavy and/or light chain framework regions and/or its heavy and/or light chain constant regions to the first immunoglobulin partner. Preferably a human antibody is the acceptor antibody.

"CDRs" are defined as the complementarity determining region amino acid sequences of an antibody which are the hypervariable regions of immunoglobulin heavy and light chains. See, e.g., Kabat et al., Sequences of Proteins of Immunological interest, 4th Ed., U.S. Department of Health and Human Services, National Institutes of Health (1987). There are three heavy chain and three light chain CDRs (or CDR regions) in the variable portion of an immunoglobulin. Thus, "CDRs" as used herein refers to all three heavy chain CDRs, or all three light chain CDRs (or both all heavy and all light chain CDRs, if appropriate).

CDRs provide the majority of contact residues for the binding of the antibody to the antigen or epitope. CDRs of interest are derived from donor antibody variable heavy and light chain sequences, and include analogs of the naturally occurring CDRs, which analogs also share or retain the same antigen binding specificity and/or neutralizing ability as the donor antibody from which they were derived.

By 'sharing the antigen binding specificity or neutralizing ability' is meant, for example, that although mAb 2B6 (see U.S. Patent Nos. 5,683,892, 5,693,323, 5,783,184, 5,851,525, and 6,129,913) may be characterized by a certain level of antigen affinity, a CDR encoded by a nucleic acid sequence of 2B6 in an appropriate structural environment may have a lower, or higher affinity. It is expected that CDRs of 2B6 in such environments will nevertheless recognize the same epitope(s) as 2B6. Exemplary heavy chain CDRs of 2B6 include SEQ ID NO: 7; SEQ ID NO: 8; SEQ ID NO: 9; and exemplary light chain CDRs of 2B6 include SEQ ID NO: 10; SEQ ID NO: 11; and SEQ ID NO:12.

A "functional fragment" is a partial heavy or light chain variable sequence (e.g., minor deletions at the amino or carboxy terminus of the immunoglobulin variable region) which retains the same antigen binding specificity and/or neutralizing ability as the antibody from which the fragment was derived.

An "analog" is an amino acid sequence modified by at least one amino acid, wherein said modification can be chemical or a substitution or a rearrangement of a few amino acids (i.e., no more than 10), which modification permits the amino acid sequence to retain the biological characteristics, e.g., antigen specificity and high affinity, of the unmodified sequence. For example, (silent) mutations can be constructed, via substitutions, when certain endonuclease restriction sites are created within or surrounding CDR-encoding regions.

Analogs may also arise as allelic variations. An "allelic variation or modification" is an alteration in the nucleic acid sequence encoding the amino acid or peptide sequences of the invention or disclosure. Such variations or modifications may be due to degeneracy in the genetic code or may be deliberately engineered to provide desired characteristics. These variations or modifications may or may not result in alterations in any encoded amino acid sequence.

The term "effector agents" refers to non-protein carrier molecules to which the altered antibodies, and/or natural or synthetic light or heavy chains of the donor antibody or other fragments of the donor antibody may be associated by conventional means. Such non-protein carriers can include conventional carriers used in the diagnostic field, e.g., polystyrene or other plastic beads, polysaccharides, e.g., as used in the BIAcore [Pharmacia] system, or other non-protein substances useful in the medical field and safe for administration to humans and animals. Other effector agents may include a macrocycle, for chelating a heavy metal atom, or radioisotopes. Such effector agents may also be useful to increase the half-life of the altered antibodies, e.g., polyethylene glycol.

"Polypeptide" refers to any peptide or protein comprising two or more amino acids joined to each other by peptide bonds or modified peptide bonds, *i.e.*, peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques that are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter, et al., "Analysis for protein modifications and nonprotein cofactors", Meth. Enzymol. (1990) 182:626-646 and Rattan, et al., "Protein Synthesis: Posttranslational Modifications and Aging", Ann NY Acad Sci (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

As used herein, "reduce" or "reducing" eosinophils refers to a decrease in the amount of eosinophils observed in the blood of a patient after administration at least one anti-IL-5 antibody.

As used herein "co-administration" or "co-administering" as used herein refers to administration of two or more compounds to the same patient. Co-administration of such compounds may be simultaneous or at about the same time (e.g., within the same hour) or it may be within several hours, days, week, or months of one another, depending on the dosing schedule of each compound. For example, a first compound may be administered once weekly while a second compound is co-administered daily. By way of another example, compounds may be co-administered if one compound is administered daily and the other compound is administered once every three months.

As used herein "maximum plasma concentration" or "Cmax" means the highest observed concentration of a substance (for example, at least one anti-IL-5 antibody) in mammalian plasma after administration of the substance to the mammal.

As used herein "Area Under the Curve" or "AUC" is the area under the curve in a plot of the concentration of a substance in plasma against time. AUC can be a measure of the integral of the instantaneous concentrations during a time interval and has the units mass*time/volume. AUC is typically calculated by the trapezoidal method (e.g., linear, linear-log). AUC is usually given for the time interval zero to infinity, and other time intervals are indicated (for example AUC (t1,t2) where t1 and t2 are the starting and finishing times for the interval). Thus, as used herein "AUC₀₋₂₄" refers to an AUC over a 24 hour period and AUC(0-inf) refers to AUC from over an infinite time period.

As used herein "Tmax" refers to the observed time for reaching the maximum concentration of a substance in plasma of a mammal after administration of that substance to the mammal.

As used herein "serum or plasma half life" refers to the time required for half the quantity of a substance administered to a mammal to be metabolized or eliminated from the serum or plasma of the mammal by normal biological processes.

As used herein "disorder associated with excess eosinophil production" means any disorder or disease in which atypical symptoms may manifest due to the production of eosinophils. Disorders associated with excess eosinophil production include but are not limited to, atopic asthma, atopic dermatitis, allergic rhinitis, non-allergic rhinitis, asthma, severe asthma, chronic eosinophilic pneumonia, allergic bronchopulmonary aspergillosis, coeliac disease, Churg-Strauss syndrome (periarteritis nodosa plus atopy), eosinophilic myalgia syndrome, hypereosinophilic syndrome, oedematous reactions including episodic angiodema, helminth infections, where eosinophils may have a protective role, onchocercal dermatitis and Eosinophil-Associated Gastrointestinal Disorders, including but not limited to, eosinophilic oesophagitis, eosinophilic gastritis, eosinophilic gastroenteritis, eosinophilic enteritis and eosinophilic colitis, nasal micropolyposis and polyposis, aspirin intolerance, asthma and obstructive sleep apnoe. Eosinophil-derived secretory products have also been associated with the promotion of angiogenesis and connective tissue formation in tumours and the fibrotic responses seen in conditions such as chronic asthma, Crohn's disease, scleroderma and endomyocardial fibrosis (Munitz A, Levi-Schaffer F. Allergy 2004; 59: 268-75, Adamko et al. Allergy 2005; 60: 13-22, Oldhoff, et al. Allergy 2005; 60: 693-6).

The therapeutic response induced by the methods of this invention and disclosure is produced by the binding of an anti-IL-5 antibody to human IL-5 and thus subsequently blocking eosinophil stimulation. Thus, the methods of the present invention and disclosure are highly desirable for those persons experiencing an allergic and/or atopic response, or a response associated with eosinophilia.

In one embodiment of the present invention and disclosure, methods are provided for reducing eosinophils in a human in need thereof, which method comprises administering to said human a composition comprising at least one anti-IL-5 antibody, wherein at least one anti-IL-5 antibody provides a mean maximum plasma concentration of said anti-IL-5 antibody of at least about 1.03 ± 0.21 µg/mL and an Area Under the Curve_{(0-inf}) value of said anti-IL-5 antibody is at least about 15.5 ± 2.7 µg×day/mL. The maximum plasma concentration may be in the range of about 12.1 ± 2.4 µg/mL to about 278 ±29 µg/mL. The AUC_{(0-inf)} may be in the range of about 207 ±34 µg×day/mL to about 4361 ± 168 µg×day/mL. Furthermore said at least one anti-IL-5 antibody has serum half-life of about 16.2 ± 2.1 days to about 21.7 ± 2.8 days.

In another aspect, at least one anti-IL-5 antibody is to human IL-5. In another aspect, at least one anti-IL antibody is neutralizing. The anti-IL-5 antibody of the present invention and/or disclosure may be humanized, fully human, murine or a fragment of any anti-IL-antibody thereof. In one aspect of the present invention, at least one anti-IL-5 antibody comprises a heavy chain of SEQ ID NO: 19 and at least one anti-lL-5 antibody comprises a light chain having SEQ ID NO: 21.

In another aspect of the present invention and disclosure, the human is suffering from a disorder associated with excess eosinophil production selected from the group consisting of atopic asthma, atopic dermatitis, allergic rhinitis, non-allergic rhinitis, asthma, severe asthma, chronic eosinophilic pneumonia, allergic bronchopulmonary aspergillosis, coeliac disease, Churg-Strauss syndrome, eosinophilic myalgia syndrome, hypereosinophilic syndrome, oedematous reactions including episodic angiodema, helminth infections, onchocercal dermatitis eosinophilic oesophagitis, eosinophilic gastritis, eosinophilic gastroenteritis, eosinophilic enteritis, eosinophilic colitis, nasal micropolyposis, nasal polyposis, aspirin intolerance asthma, obstructive sleep apnoe, chronic asthma, Crohn's disease, scleroderma and endomyocardial fibrosis.

In another aspect of the present invention and disclosure the composition comprising at least one anti-IL-5 antibody is administered subcutaneously, which may be at a dose of 250 mg. A subcutaneous dose may be administered one to three times or more to a human. Mean plasma concentration of said at least one anti-IL-5 antibody from a subcutaneous injection may be about 34.1 ± 12.1 µg/mL to about 38.2 ± 9.1 µg/mL. AUC_{(0-inf)} of said at least one anti-IL-5 antibody from a subcutaneous injection may be about 1110 ± 372 µg×day/mL to about 1196 ± 254 µg×day/mL.

In another aspect of the present invention and disclosure, methods are provided wherein said composition comprising an anti-IL-5 antibody is administered intramuscularly. Intramuscular injection of a composition comprising at least one anti-IL-5 antibody may be administered at a dose of 250 mg. The mean plasma concentration of said at least one anti-IL-5 antibody from intramuscular injection may be about 46.9 ± 10.6 µg/mL and the AUC_{(0-inf)} of said at least one anti-IL-5 antibody may be about 1395 ± 348 µg×day/mL.

In yet another aspect of the present invention and disclosure, methods are provided wherein said composition comprising said at least one anti-IL-5 antibody is administered intravenously. An anti-IL-5 antibody administered intravenously may be administered at a dose of 250 mg to a dose of 750 mg, which may be administered over the course of 20-60 minutes or over about 30 minutes. The mean plasma concentration of said at least one anti-IL-5 antibody administered intravenously may be about 109 ± 17.0 µg/mL and the AUC_{(0-inf)} of said at least one anti-IL-5 antibody may be about 1557 ± 250 µg×day/mL.

The present invention and disclosure also provides methods for reducing eosinophils in a human in need thereof, comprising administering a composition comprising a first anti-IL-5 antibody and a second anti-IL-5 antibody. Methods are also provided herein wherein at least one anti-IL-5 antibody is co-administered with a steroid.

Also, provided herein are methods of treating nasal polyposis, in a human, comprising the step of administering to said human in need thereof an effective amount of a composition comprising at least one anti-IL-5 antibody wherein said antibody comprises a heavy chain and a light chain. In one aspect of the present invention, the antibody comprises a heavy chain comprising SEQ ID NO: 19 and/or the antibody comprises a light chain comprising SEQ ID NO: 21. In another aspect, the nasal polyposis is severe.

In one embodiment, the size of at least one nasal polyp in said human is reduced after at least one dose of said composition comprising at least one anti-IL-5 antibody. The size of said at least one nasal polyp remains reduced for at least two months. Administration of said composition comprising at least one anti-IL-5 antibody can reduce or eliminates the need of said human for surgery for nasal polyposis.

In another aspect, the human with nasal polyps is also suffering from a disorder associated with excess eosinophil production selected from the group consisting of atopic asthma, atopic dermatitis, allergic rhinitis, non-allergic rhinitis, asthma, severe asthma, chronic eosinophilic pneumonia, allergic bronchopulmonary aspergillosis, coeliac disease, Churg-Strauss syndrome, eosinophilic myalgia syndrome, hypereosinophilic syndrome, oedematous reactions including episodic angiodema, helminth infections, onchocercal dermatitis eosinophilic oesophagitis, eosinophilic gastritis, eosinophilic gastroenteritis, eosinophilic enteritis, eosinophilic colitis, nasal micropolyposis, aspirin intolerance asthma, obstructive sleep apnoe, chronic asthma, Crohn's disease, scleroderma and endomyocardial fibrosis. The composition comprising at least one anti-IL-5 antibody comprises a first anti-IL-5 antibody and a second anti-IL-5 antibody. The composition comprising at least one anti-IL-5 antibody can be co-administered with a steroid. The at least one anti-IL-5 antibody may be administered intravenously as 750 mg over about 30 minutes.

A skilled artisan will understand the various methods for measuring and calculating the pharmacokinetic (for example, but not limited to, Cmax, AUC, Tmax, serum half-life) and pharmacodynamic (for example, but not limited to, eosinophil levels) parameters described herein. Furthermore, the skilled artisan will understand the various methods for making statistical comparisons (for example, but not limited to, comparisons of change from baseline to post-treatment and/or comparisons among treatment groups) and/or analysis of the pharmacokinetic and pharmacodynamic parameters described herein.

### Examples

The following examples illustrate various aspects of this invention and disclosure.

### Example 1 - Pharmacokinetics of an anti-IL-5 antibody in Healthy Volunteers

Pharmacokinetics in normal volunteers was evaluated following subcutaneous (sc), intramuscular (im), and intravenous (iv) administration. The objectives of this study were to estimate the bioavailability of a single 250 mg dose of mepolizumab (a humanized, anti-IL-5 antibody described herein) from three different sc sites and an im site compared with an iv dose and to make a preliminary assessment of the safety and tolerability of mepolizumab and its effect on eosinophil counts in healthy volunteers. This was an open, single dose, parallel group study. Human subjects were allocated to receive doses of mepolizumab as shown in Table 1.

**Table 1 Dose Allocation**

| **No. of Subjects** | **Dose** | **Route** | **Site of Injection** |
|---|---|---|---|
| 12 | 250 mg | Sc | Abdomen - lower anterior wall |
| 12 | 250 mg | Sc | Arm - upper outer aspect |
| 12 | 250 mg | Sc | Thigh - upper outer aspect |
| 12 | 250 mg | Im | Lateral thigh |
| 12 | 250 mg | Iv | Forearm vein |

Each human subject received one dose of study medication (mepolizumab) and was followed for 12 weeks with blood samples taken at intervals for assay of mepolizumab and eosinophil count.

Sixty healthy subjects, between 18 and 55 years of age, 23 males and 37 females were recruited to achieve 12 evaluable subjects per group. At least four of one sex were recruited to each dose group. Each 250 mg sc dose was administered as 2 x 125 mg (2 x 1ml) injections of mepolizumab, the im dose was administered as 1 x 250 mg (1 x 2ml) injection, and the iv dose was administered as an infusion over approximately 30 minutes.

Serial blood samples were collected pre-dose and nominally up to 12 weeks after administration of mepolizumab. Plasma samples were assayed for mepolizumab using an immunoassay method (Lower Limit of Quantitation [LLQ] 0.05 µg/mL for a 0.1 mL aliquot of 10% human plasma). Non-compartmental pharmacokinetic analysis was used to derive the parameters AUC(0-inf), Cmax, Tmax, and T1/2 for mepolizumab.

Following the 30 minute iv infusion, mepolizumab plasma concentrations declined in a seemingly bi-exponential manner. Tmax ranged from 0.5 to 4 hours, relative to the start of the 30 minute infusion.

Following administration of bolus sc injections at three different sites and the bolus im injection, the mean mepolizumab plasma concentration-time profiles were similar in shape. However, overall the mean plasma concentrations were higher following im administration. Mepolizumab was absorbed slowly, with Tmax values ranging between 2 and 14 days. Table 2 summarizes the PK parameters for mepolizumab.

**Table 2 Mean (SD) Pharmacokinetic Parameters for Mepolizumab Following Administration at 3 Subcutaneous Sites, 1 Intramuscular Site, and Intravenous Administration of 250 mg**

| | | SC | | | |
|---|---|---|---|---|---|
| | Abdomen (n = 12) | Arm (n = 12) | Thigh (n = 12) | IM (n = 12) | IV (n = 12) |
| AUCO_{0-inf}, µg×day/mL | 1110 ± 372 | 1238 ± 228 | 1196 ± 254 | 1395 ± 348 | 1557 ± 250 |
| C_{max,} µg/mL | 34.1 ± 12.1 | 34.9 ± 7.3 | 38.2 ± 9.1 | 46.9 ± 10.6 | 109 ± 17.0 |
| Tₘₐₓ, days^{a} | | 5(3-14) | 5(2-7) | 4(3-7) | 0.08 (0.02-0.2) |
| T_{½}, days | 7(4-14) | 20.4 ± 2.6 | 18.5 ± 3.5 | 19.2 ± 4.2 | 18.5 ± 2.3 |
| | 17.9 ± 3.3 | | | | |

| | | | | | |
|---|---|---|---|---|---|
| a Median (range). AUC_{0-inf} = area under plasma concentration-time curve from 0 to infinity; Cₘₐₓ = maximum plasma concentration; IM = intramuscular; IV = intravenous; SC = subcutaneous; Tₘₐₓ = time to Cₘₐₓ; T_{½}= terminal half life. | | | | | |

### Example 2 - Patients with Mild to Moderate Atopic Asthma

A multicentre, double-blind, randomized, placebo-controlled study was conducted in men, 18-46 years of age, with mild atopic asthma (forced expiratory volume in 1 second [FEV₁] ≥70% predicted and on β₂-agonists). The starting dose used in this study (0.05 mg/kg) was the lowest dose administered to cynomolgus monkeys (0.05, 0.5, 5 or 50 mg/kg IV; n = 2/sex/group) in a two-dose toxicity study; the other doses were based on the lowest dose in the same toxicity study at which a >85% decrease in basal eosinophil counts was observed (i.e. 5 mg/kg). Pharmacokinetic parameters were assessed after a single, 30-minute IV infusion of mepolizumab 0.05 mg/kg (n = 4), 0.5 mg/kg (n = 5), 2.5 mg/kg (n = 8) or 10 mg/kg (n = 8) (Table 3). Plasma mepolizumab concentrations declined bi-exponentially after the infusion, Cₘₐₓ (mean ± SD) ranged from 1.03 ± 0.21 µg/mL at the 0.05 mg/kg dose level to 215 ± 28 µg/mL at the 10 mg/kg dose level and Tₘₐₓ occurred at 0.5-3 hours. Both plasma clearance and volume of distribution at steady state were similar across the dose range, indicating linear pharmacokinetics. There was little inter-subject variability in the area under the plasma concentration-time curve (AUC) or Cₘₐₓ (coefficients of variation were generally <20%). The mean (± SD) terminal half-life was relatively constant across doses, ranging from 19.0 ± 2.5 days with 10 mg/kg to 20.0 ± 1.9 days with 0.5 mg/kg, and mepolizumab was quantifiable in plasma for up to 16 weeks post-dose in the majority of subjects. (Table 3) In terms of pharmacodynamics, mepolizumab was associated with a persistent dose-dependent decrease in peripheral eosinophils, which was apparent at all doses and was maintained until the final follow-up visit at Week 16 in patients on the 10 mg/kg dose.

**Table 3. Pharmacokinetic parameters (mean ± SD) for mepolizumab following a single intravenous infusion in men with mild atopic asthma**

| | Mepolizumab dose (mg/kg) | | | |
|---|---|---|---|---|
| | 0.05 (n = 4) | 0.5 (n = 5) | 2.5 (n = 8) | 10 (n = 8) |
| AUC_{0-inf}, µg×day/mL | 15.5 ± 2.7 | 168 ± 19 | 846 ± 164^{a} | 3345 ± 324 |
| Cₘₐₓ µg/mL | 1.03 ± 0.21 | 10.6 ± 2.2 | 51.4 ± 7.9^{a,b} | 215 ± 28 |
| T_{½}, days | 19.7 ± 7.6 | 20.0 ± 1.9 | 19.3 ± 2.9 | 19.0 ± 2.5 |
| Plasma clearance, mL/h/kg | 0.137 ± 0.022 | 0.125 ± 0.014 | 0.129 ± 0.023 | 0.126 ± 0.013 |
| Steady state volume of distribution, mL/kg | 85.0 ± 19.8 | 82.2. ± 14.5 | 80.6 ± 7.8 | 78.9 ± 11.0 |

| | | | | |
|---|---|---|---|---|
| a Data from one human subject excluded, due to incorrect dosing. b Cₘₐₓ value for one subject excluded, as it was approximately 4-5-fold higher than other values in the cohort. AUC_{0-inf} = area under plasma concentration-time curve from 0 to infinity; Cₘₐₓ = maximum plasma concentration; T_{½} = terminal half life. | | | | |

Similar pharmacokinetics were reported in a further double-blind, randomized, placebo-controlled study in men, 18-43 years of age, with mild asthma (FEV₁ >64% predicted and on β₂-agonists with or without inhaled corticosteroids at 400-1000 µg/day) who received a single IV infusion of mepolizumab 0.5 mg/kg (n = 4), 2.5 mg/kg (n = 4) or 10 mg/kg (n = 4). A persistent and dose-dependent reduction in peripheral eosinophil count relative to baseline was observed in most patients treated with mepolizumab (estimated maximal decrease of ∼85%). The duration of eosinophil suppression increased with increasing dose, the maximal reduction in eosinophil count occurring approximately 3-4 days after Cₘₐₓ.

The pharmacokinetics of mepolizumab following multiple dosing were also evaluated in patients with asthma as part of a multicentre, double-blind, randomized, placebo-controlled, parallel-group study. Three IV infusions of mepolizumab 250 mg (n = 120) or 750 mg (n = 116) or placebo (n = 126) were administered at intervals of 1 month to patients, 18-55 years of age, with persistent mild-to-moderate asthma (FEV₁ 50-80% predicted, managed with inhaled beclomethasone dipropionate ≤1000 µg/day or equivalent). Dose-proportional and time-independent pharmacokinetics were observed with repeat dosing in this population. In addition, both mepolizumab doses reduced blood eosinophils by approximately 80% from baseline (p < 0.001 vs placebo). This decrease was apparent 1 week after the first infusion and was maintained for 12 weeks after the last infusion (to Week 20). Induced sputum was examined in a subgroup of 37 patients. This showed that sputum eosinophil numbers also decreased with mepolizumab 250 mg and 750 mg, starting 4 weeks after the first infusion and persisting to the final follow-up visit at Week 20; the reduction from baseline to Week 12 was statistically significant for mepolizumab 750 mg (p = 0.013). Neither circulating nor sputum eosinophil levels changed significantly in the placebo group. Interestingly, the profound decrease in the number of eosinophils in the blood was not accompanied by a parallel reduction in bronchial mucosa or bone marrow eosinophils: while the reduction in blood was approximately 80% from baseline, the reduction in bronchial mucosa was approximately 50% from baseline. It is possible that airway eosinophils are less dependent upon IL-5, or that depletion of eosinophils in the bronchial mucosa may require a longer period of treatment.

Time-independent pharmacokinetics were also observed in a double-blind, placebo-controlled, parallel-group study after repeated SC administration of mepolizumab. Men and women, aged 19-50 years, with mild asthma received three SC doses of mepolizumab 250 mg (n = 8) or placebo (n = 8). The first two doses were given 6 weeks apart and the third dose was given 2 weeks

Mepolizumab was quantifiable for up to 16 weeks post-dosing in the majority of human subjects receiving a single iv dose. Two subjects had quantifiable concentrations at time zero that were less than 1 % of the Cmax values observed in these subjects. These quantifiable pre-dose concentrations were set to zero for the computation of AUC(0-inf).

One other subject received a dose of approximately 0.0877 mg/kg instead of 2.5 mg/kg. AUC_{(0-inf)}, Cmax, and Tmax data from this subject were excluded and CL and Vss values for this subject were calculated using the actual dose of 0.0877 mg/kg.

For the compartmental analysis, a two-compartment model appeared to fit the concentration-time profiles reasonably well, with the exception of the 0.05 mg/kg dose in which the number of data points was limited due to sparse sampling and non-quantifiable concentrations. Goodness of fit was evidenced by the generally low standard errors (Percent Coefficient of Variation [CV%]<35%). The AUC(0-inf), CL; and Vss data generated by the compartmental analysis were also generally in close agreement with data from the non-compartmental analysis. The majority of the area under the plasma concentration versus time curve (generally >90%) was associated with the terminal phase. The initial and terminal phase half-life values were approximately 2 and 20 days, respectively.

### Example 3 - Comparison of Single IV infusion in human patients with Mild Asthma

Following administration of 0.5 to 10 mg/kg doses as a single 30 minute iv infusion to males with mild asthma, mepolizumab exhibited dose-proportional PK and a long elimination half-life of approximately 20 days (Table 4). Plasma clearance and steady-state volume of distribution were relatively constant across the dose range studied (Table 4). The inter-subject variability in PK parameters of mepolizumab was low (CV% values of 20% or less).

**Table 4 Mean (SD) Pharmacokinetic Parameters for Mepolizumab Following a Single Thirty-minute Infusion of Mepolizumab to Males with Mild Asthma**

| | **Dose Range (mg/kg)** | | |
|---|---|---|---|
| **Parameter** | **0.5 mg/kg n=4** | **2.5 mg/kg n=4** | **10 mg/kg n=4** |
| AUC(0-inf) (µg. day/mL) | 207 (34) | 1327 (247) | 4361 (168) |
| Cmax (µg/mL) | 12.1 (2.4) | 79.0 (4.3) | 278 (29) |
| CL (mL/h/kg) | 0.103 (0.017) | 0.081 (0.015) | 0.096 (0.004) |
| Vss (mL/kg) | 68.4 (2.5) | 55.4 (5.2) | 59.3 (3.7) |
| T1/2 (days) | 20.9 (4.0) | 21.7 (2.8) | 20.9 (2.6) |

### Example 4 - Subcutaneous doses in Patients with Mild Asthma

Three sc doses of 250 mg were administered to patients with mild asthma using the following regimen: the first and second doses were separated by six weeks with the second and third doses separated by two weeks. The data indicate that mepolizumab has a bioavailability of approximately 50% following sc injections into the lateral abdominal wall as compared with iv infusion administered to another group of patients. As expected, based on the short time separation between the three doses (6 and 2 weeks) relative to the long terminal half-life of mepolizumab (20 days), the mean AUC_{(o-inf)} and Cmax were approximately 65% and 80%, respectively, higher after the third dose compared with after the first dose. The drug was absorbed slowly with Tmax values ranging between approximately 2 and 14 days. Mean PK parameters for mepolizumab are summarized in Table 5.

**Table 5 Mean (SD) Pharmacokinetic Parameters for Mepolizumab Following Single or Repeated Subcutaneous Administration of 250 mg Mepolizumab**

| **Parameter (units)** | **Dose 1 (N=8)** | **Dose 3 (N=8)** |
|---|---|---|
| AUC(0-inf) (µg.d/mL) | 560 (197) | 924 (189) |
| Cmax | 17.7 (7.1) | 32.2 (7.8) |
| Tmax (days) | 4.50 (3.00-7.02) | 8.01 (2.02-13.96) |
| T½ (days) | 20.5 (5.3) | 16.2 (2.1) |

| | | |
|---|---|---|
| 1. Data presented as median (range). | | |

Individual concentration-time profiles following the first dose administration were fitted to a one-compartment model with first order absorption. The mean parameter values were used to simulate a concentration-time profile following administration of the dosing regimen employed in this study. The predicted average concentrations following the third dose fall within the range of observed concentrations, suggesting time independent pharmacokinetics.

### Example 5

Following three monthly administrations of either 250 mg or 750 mg iv infusions, plasma concentrations of mepolizumab were generally quantifiable at all study visits. Based on these preliminary data, mean plasma concentrations of mepolizumab at each visit increased in an approximately dose-proportional manner between the 250 and 750 mg doses (Table 6). The means of the actual concentrations at each visit were similar to the predicted multiple-dose concentration data which were simulated using a 2-compartment iv infusion model and pharmacokinetic parameters estimated from previous single-dose data (Table 6). Thus, mepolizumab exhibits dose-proportional and time-independent pharmacokinetics.

**Table 6 Mean (SD) Plasma Mepolizumab Concentrations (microgram/mL) by Dose and Visit**

| | **Visit 3 Post-Infusion** | **Visit 4** | **Visit 5** | **Visit 6 Pre-dose** | **Visit 8 Pre-dose** | **Visit 8 Post-dose** | **Visit 10** | **Visit 11** | **Visit 12** |
|---|---|---|---|---|---|---|---|---|---|
| **Days** | **1** | **8 ± 2** | **15 ± 2** | **29 ± 2** | **57 ± 7** | **57 ± 7** | **85t7** | **113±7** | **141±7** |
| **Dose (mg)** | n=55 | n=53 | n=51 | n=51 | n=86 | n=49 | n=36 | n=32 | n=25 |
| **250** | 90.0 (33.2) | 34.8 (15.4) | 23.1 (6.4) | 14.0 (11.5) | 18.9 (8.1) | 115 (40) | 22.2 (9.1) | 7.03 (3.79) | 3.17 (1.77) |
| | | | | | | | | | |

| | **Visit 3 Post Infusion** | **Visit 4** | **Visit 5** | **Visit 6 Pre-dose** | **Visit 8 Pre-dose** | **Visit 8 Post-dose** | **Visit 10** | **Visit 11** | **Visit 12** |
|---|---|---|---|---|---|---|---|---|---|
| **Days** | 1 | **8 ± 2** | **15 ± 2** | **29 ± 2** | **57 ± 7** | **57±7** | **85±7** | **113±7** | **141±7** |
| **Dose (mg)** | n=47 | n=46 | n=45 | n=42 | n=84 | n=53 | n=47 | n=42 | n=27 |
| **750** | 221 (60) | 90.9 (34.3) | 60.2 (15.5) | 34.5 (9.9) | 49.0 (14.1) | 254 (74) | 51.2 (19.5) | 18.2 (7.8) | 7.04 (3.39) |

| | | | | | | | | | |
|---|---|---|---|---|---|---|---|---|---|
| Note: The number of days for visits 4 to 12 were days relative to the first dose of mepolizumab. | | | | | | | | | |

### Example 6 - Human Patients with Hypereosiniphilic Syndrome

Following 9 monthly administrations (0 to 32 weeks) of 750 mg iv infusions, plasma concentrations of mepolizumab were quantifiable at all study visits (from post-dose Day 1) for all human patients who had blood samples drawn, except at one visit for one patient. Based on preliminary data, the mean concentration values were similar to those in the previous examples presented above following 750 mg of mepolizumab by IV injection over aout 30 minutes (Table 7). The means of the actual concentrations at each visit were similar to the predicted multiple-dose concentration data which were simulated using a 2-compartment iv infusion model and pharmacokinetic parameters estimated from previous single-dose data (Table 7). Thus, mepolizumab pharmacokinetics in patients with HES are similar to those previously seen.

**Table 7 Mean (SD) Mepolizumab Plasma Concentration by Week**

| **Week (Day range)^{1,2}** | **N** | **Mean (SD) [µg/mL]** | **CV%** |
|---|---|---|---|
| Week 0 (1 post-infusion) | 37 | 225 (74.7) | 33 |
| Week 1 (6-10) | 38 | 85.0 (23.8) | 28 |
| Week 2 (13-17) | 36 | 58.0 (17.7) | 31 |
| Week 3 (19-24) | 35 | 41.5 (16.9) | 41 |
| Week 4 (25-37) | 44 | 31.4 (13.7) | 44 |
| Week 8 (54-65) | 38 | 43.6 (20.1) | 46 |
| Week 16 (110-134) Pre-infusion | 36 | 44.5 (24.9) | 56 |
| Week 16 (110-134) Post-infusion | 37 | 263 (91.3) | 35 |
| Week 24 (164-196) | 32 | 57.6 (40.6) | 71 |
| Week 32 (221-232) | 30 | 48.3 (30.8) | 64 |
| Week 36 (239-266) | 29 | 53.7 (26.0) | 49 |
| Post Week 36 (267-361) | 4 | 31.4 (22.3) | 71 |

| | | | |
|---|---|---|---|
| 1. The range of days relative to the first dose of mepolizumab. 2. The two quantifiable pre-dose values are not included. | | | |

### Example 7 - Pharmacokinetic/Pharmacodynamic Relationships

Following administration of 0.5 to 10 mg/kg iv mepolizumab, a persistent and dose-dependent reduction in peripheral eosinophil count relative to baseline was observed in the majority of patients who received mepolizumab. The duration of the suppression in cell count increased with increasing dose. Maximal depression in cell count occurred approximately 3 to 4 days after maximum plasma concentrations were achieved. The relationship between % baseline eosinophil counts and plasma concentrations of mepolizumab was well described using an indirect pharmacologic response model with inhibition of the synthesis (production) rate constant. The estimated maximal decrease in eosinophil count following administration of mepolizumab was approximately 85% from baseline and the concentration of drug resulting in half-maximal effect on eosinophil count (IC₅₀) was approximately 0.4 µg/mL.

### Example 8 - Human Patients with Nasal Polyps

**Rational:** 90% of nasal polyps are characterised by prominent eosiophilia. IL-5 is key in eosinophilic differentiation and survival and its antagonism is a potential novel treatment strategy for patients with nasal polyps.

**Methods:** 30 subjects with severe nasal polyposis (grades 3-4) were randomized in double blind fashion to receive either 2 single IV injections (28 days apart) of 750mg of mepolizumab, a humanized anti-interleukin-5 monoclonal antibody (n=20), or placebo (n=10). Changes in nasal polyp score and in comparative nasal polyp score were assessed relative to baseline (week 0) at 1 and 2 months post last dose (week 8 and 12). A 1 point reduction in these endoscopic assessments is considered clinically significant.

**Results:** No differences in baseline values were observed between mepolizumab and placebo. A significant reduction was observed in nasal polyp score with Mepolizumab vs placebo at week 8 (60% vs 10%, p=0.011) and week 12 (65% vs 20%, p=0.025). 65% subjects on mepolizumab showed a 'much better' or 'better' improvement of comparative nasal polyp score compared to 10% on placebo by week 8 and 70% on mepolizumab vs 20% on placebo at week 12. Over 50% of subjects on mepolizumab demonstrated improvement on blinded assessment of CT scans by three independent observers by week 8. The requirement for surgery was also reduced in the group on mepolizumab with 15% subjects requesting surgery in the group on mepolizumab vs 50% in the group on placebo by week 12. Individual response and magnitude of response is maintained across the endpoints analysed.

**Conclusion:** Two doses of mepolizumab 750mg iv reduces the size and volume of for at least 2 months post first dose. IL-5 antagonism is a potential novel therapeutic alternative for subjects with severe polyposis.

## Claims

1. An anti-IL-5 antibody for use in reducing eosinophils in a human suffering from hypereosinophilic syndrome, wherein said anti-IL-5 antibody is to be administered to said human in a composition which comprises said at least one anti-IL-5 antibody, wherein said anti-IL-5 antibody comprises a heavy chain comprising SEQ ID NO: 19 and a light chain comprising SEQ ID NO: 21 and provides a mean maximum plasma concentration of said anti-IL-5 antibody of at least 1.03 ± 0.21 µg/mL to 278 ±29 µg/mL and an Area Under the Curve_{(0-inf)} value of said anti-IL-5 antibody is at least 15.5 ± 2.7 µg×day/mL to 4361 ±168 µg×day/mL.

2. The anti-IL-5 antibody for the use as claimed in claim 1, wherein said mean maximum plasma concentration is in the range of 12.1 ± 2.4 µg/mLto 278 ±29 µg/mLand/ or wherein said AUC_{(0-inf)} is in the range of 207 ±34 µg×day/mL to 4361 ± 168 µg×day/mL.

3. The anti-IL-5 antibody for the use as claimed in claim 1 or claim 2, wherein said composition comprising at least one anti-IL-5 antibody is to be administered subcutaneously.

4. The anti-IL-5 antibody for the use as claimed in claim 3, wherein said composition comprising the antibody is to be administered at a dose of 250 mg.

5. The anti-IL-5 antibody for the use as claimed in claim 4, wherein said subcutaneous dose is to be administered one to three times.

6. The anti-IL-5 antibody for the use as claimed in claim 5, wherein the mean plasma concentration of said antibody is 34.1 ± 12.1 µg/mL to 38.2 ± 9.1 µg/mLand/or wherein the AUC_{(0-inf)} of said antibody is 1110 ± 372 µg×day/mL to 1196 ± 254 µg×day/mL.

7. The anti-IL-5 antibody for the use as claimed in claim 1, wherein said composition comprising the antibody is to be administered intramuscularly.

8. The anti-IL-5 antibody for the use as claimed in claim 7, wherein said composition comprising the antibody is administered at a dose of 250 mg.

9. The anti-IL-5 antibody for the use as claimed in claim 8, wherein said the mean plasma concentration of said antibody is 46.9 ± 10.6 µg/mL and the AUC_{(0-inf)} of said antibody is 1395 ± 348 µg×day/mL.

10. The anti-IL-5 antibody for the use as claimed in claim 1, wherein said composition comprising the antibody is to be administered intravenously.

11. The anti-IL-5 antibody for the use as claimed in claim 10, wherein said composition comprising the antibody is to be administered at a dose of 250 mg or at a dose of 750 mg.

12. The anti-IL-5 antibody for the use as claimed in claim 10 or 11, wherein the antibody is administered over about a 30 minute infusion.

13. The anti-IL-5 antibody for the use as claimed in claim 10, wherein said the mean plasma concentration of said antibody is 109 ± 17.0 µg/mL and the AUC_{(0-inf)} of said antibody is 1557 ± 250 µg×day/mL.

14. The anti-IL-5 antibody for the use as claimed in any one of claims 1 to 13, wherein said antibody has serum half-life of 16.2 ± 2.1 days to 21.7 ± 2.8 days.

15. The anti-IL-5 antibody for the use as claimed in any one of claims 1 to 14, wherein said composition comprising the antibody is co-administered with a steroid.

## Patentansprüche

1. Anti-IL-5-Antikörper zur Verwendung bei der Reduzierung von Eosinophilen bei einem Menschen, der an hypereosinophilem Syndrom leidet, wobei der Anti-IL-5-Antikörper dem Menschen in einer Zusammensetzung zu verabreichen ist, die den mindestens einen Anti-IL-5-Antikörper umfasst, wobei der Anti-IL-5-Antikörper eine schwere Kette umfassend SEQ ID NO:19 und eine leichte Kette umfassend SEQ ID NO:21 umfasst und eine mittlere Plasmahöchstkonzentration des Anti-IL-5-Antikörpers von mindestens 1,03 ± 0,21 µg/ml bis 278 ± 29 µg/ml bereitstellt und der AUC_{(0-inf)}-Wert für die Fläche unter der Kurve des Anti-IL-5-Antikörpers mindestens 15,5 ± 2,7 µg x Tag/ml bis 4361 ± 168 µg x Tag/ml ist.

2. Anti-IL-5-Antikörper zur Verwendung nach Anspruch 1, wobei die mittlere Plasmahöchstkonzentration im Bereich von 12,1 ± 2,4 µg/ml bis 278 ± 29 µg/ml liegt und/oder wobei der AUC_{(0-inf)}-Wert im Bereich von 207 ± 34 µg x Tag/ml bis 4361 ± 168 µg x Tag/ml liegt.

3. Anti-IL-5-Antikörper zur Verwendung nach Anspruch 1 oder Anspruch 2, wobei die Zusammensetzung, die mindestens einen Anti-IL-5-Antikörper umfasst, subkutan zu verabreichen ist.

4. Anti-IL-5-Antikörper zur Verwendung nach Anspruch 3, wobei die den Antikörper umfassende Zusammensetzung in einer Dosis von 250 mg zu verabreichen ist.

5. Anti-IL-5-Antikörper zur Verwendung nach Anspruch 4, wobei die subkutane Dosis ein- bis dreimal zu verabreichen ist.

6. Anti-IL-5-Antikörper zur Verwendung nach Anspruch 5, wobei die mittlere Plasmakonzentration des Antikörpers 34,1 ± 12,1 µg/ml bis 38,2 ± 9,1 µg/ml ist und/oder der AUC_{(0-inf)}-Wert des Antikörpers 1110 ± 372 µg x Tag/ml bis 1196 ± 254 µg x Tag/ml ist.

7. Anti-IL-5-Antikörper zur Verwendung nach Anspruch 1, wobei die den Antikörper umfassende Zusammensetzung intramuskulär zu verabreichen ist.

8. Anti-IL-5-Antikörper zur Verwendung nach Anspruch 7, wobei die den Antikörper umfassende Zusammensetzung in einer Dosis von 250 mg zu verabreichen ist.

9. Anti-IL-5-Antikörper zur Verwendung nach Anspruch 8, wobei die mittlere Plasmakonzentration des Antikörpers 46,9 ± 10,6 µg/ml ist und der AUC_{(0-inf)}-Wert des Antikörpers 1395 ± 348 µg x Tag/ml ist.

10. Anti-IL-5-Antikörper zur Verwendung nach Anspruch 1, wobei die den Antikörper umfassende Zusammensetzung intravenös zu verabreichen ist.

11. Anti-IL-5-Antikörper zur Verwendung nach Anspruch 10, wobei die den Antikörper umfassende Zusammensetzung in einer Dosis von 250 mg oder in einer Dosis von 750 mg zu verabreichen ist.

12. Anti-IL-5-Antikörper zur Verwendung nach Anspruch 10 oder 11, wobei der Antikörper mittels einer etwa 30-minütigen Infusion verabreicht wird.

13. Anti-IL-5-Antikörper zur Verwendung nach Anspruch 10, wobei die mittlere Plasmakonzentration des Antikörpers 109 ± 17,0 µg/ml ist und der AUC_{(0-inf)}-Wert des Antikörpers 1557 ± 250 µg x Tag/ml ist.

14. Anti-IL-5-Antikörper zur Verwendung nach einem der Ansprüche 1 bis 13, wobei der Antikörper eine Serumhalbwertszeit von 16,2 ± 2,1 Tagen bis 21,7 ± 2,8 Tagen hat.

15. Anti-IL-5-Antikörper zur Verwendung nach einem der Ansprüche 1 bis 14, wobei die den Antikörper umfassende Zusammensetzung zusammen mit einem Steroid verabreicht wird.

## Revendications

1. Anticorps anti-IL-5 pour une utilisation dans la réduction des éosinophiles chez un humain souffrant du syndrome hyperéosinophilique, dans lequel ledit anticorps anti-IL-5 doit être administré audit humain dans une composition comprenant ledit au moins un anticorps anti-IL-5, dans lequel ledit anticorps anti-IL-5 comprend une chaîne lourde comprenant SEQ ID NO : 19 et une chaîne légère comprenant SEQ ID NO : 21 et fourni une concentration plasmatique maximale moyenne dudit anticorps anti-IL-5 d'au moins 1,03 ± 0,21 µg/ml à 278 ± 29 µg/ml et la valeur de surface sous la courbe_{(0-inf)} dudit anticorps anti-IL-5 est au moins de 15,5 ± 2,7 µg×jour/ml à 4361 ± 168 µg×jour/ml.

2. Anticorps anti-IL-5 pour l'utilisation selon la revendication 1, dans lequel ladite concentration plasmatique maximale moyenne est située dans la plage allant de 12,1 ± 2,4 µg/ml à 278 ± 29 µg/ml et/ou dans lequel ladite SSC_{(0-inf)} est située dans la plage allant de 207 ± 34 µg×jour/ml à 4361 ± 168 µg×jour/ml.

3. Anticorps anti-IL-5 pour l'utilisation selon la revendication 1 ou la revendication 2, dans lequel ladite composition comprenant au moins un anticorps anti-IL-5 doit être administrée par voie sous-cutanée.

4. Anticorps anti-IL-5 pour l'utilisation selon la revendication 3, dans lequel ladite composition comprenant l'anticorps doit être administrée à une dose de 250 mg.

5. Anticorps anti-IL-5 pour l'utilisation selon la revendication 4, dans lequel ladite dose sous-cutanée doit être administrée une à trois fois.

6. Anticorps anti-IL-5 pour l'utilisation selon la revendication 5, dans lequel la concentration plasmatique moyenne dudit anticorps est de 34,1 ± 12,1 µg/ml à 38,2 ± 9,1 µg/ml et/ou dans lequel la SSC_{(0-inf)} dudit anticorps est de 1110 ± 372 µg×jour/ml à 1196 ± 254 µg×jour/ml.

7. Anticorps anti-IL-5 pour l'utilisation selon la revendication 1, dans lequel ladite composition comprenant l'anticorps doit être administrée par voie intramusculaire.

8. Anticorps anti-IL-5 pour l'utilisation selon la revendication 7, dans lequel ladite composition comprenant l'anticorps est administrée à une dose de 250 mg.

9. Anticorps anti-IL-5 pour l'utilisation selon la revendication 8, dans lequel ladite concentration plasmatique moyenne dudit anticorps est de 46,9 ± 10,6 µg/ml et la SSC_{(0-inf)} dudit anticorps est de 1395 ± 348 µg×jour/ml.

10. Anticorps anti-IL-5 pour l'utilisation selon la revendication 1, dans lequel ladite composition comprenant l'anticorps doit être administrée par voie intraveineuse.

11. Anticorps anti-IL-5 pour l'utilisation selon la revendication 10, dans lequel ladite composition comprenant l'anticorps doit être administrée à une dose de 250 mg ou à une dose de 750 mg.

12. Anticorps anti-IL-5 pour l'utilisation selon la revendication 10 ou 11, dans lequel l'anticorps est administré pendant environ 30 minutes par perfusion.

13. Anticorps anti-IL-5 pour l'utilisation selon la revendication 10, dans lequel ladite concentration plasmatique moyenne dudit anticorps est de 109 ± 17,0 µg/ml et la SSC_{(0-inf)} dudit anticorps est de 1557 ± 250 µg×jour/ml.

14. Anticorps anti-IL-5 pour l'utilisation selon l'une quelconque des revendications 1 à 13, dans lequel la demi-vie sérique dudit anticorps est de 16,2 ± 2,1 jours à 21,7 ± 2,8 jours.

15. Anticorps anti-IL-5 pour l'utilisation selon l'une quelconque des revendications 1 à 14, dans lequel ladite composition comprenant l'anticorps est co-administrée avec un stéroïde.
